# EUROPEAN PATENT APPLICATION

(11) **EP 4 772 107 A1**
(43) Date of publication of application: **08.07.2026**
(21) Application number: 25221404.4
(22) Date of filing: 08.12.2025
(51) Int. Cl.: A61B 5/00, A61B 5/024, A61B 5/0507, A61B 5/145

(54) **MILLIMETER WAVE RADAR BLOOD-ALCOHOL CONTENT DETECTION SYSTEM AND METHOD**

(30) Priority: 02.01.2025 US 202519008018
(71) Applicant: Honeywell International Inc., Charlotte, NC 28202 (US)
(72) Inventor: FINSETH, Tor, Charlotte, 28202 (US); LUBOLD, Nichola, Charlotte, 28202 (US)
(74) Representative: Ingrassia, Fisher & Lorenz UK Ltd.

(57) **Abstract**

A system and method for detecting the blood-alcohol content of a person includes detecting, using a millimeter-wave (mmWave) radar sensor, at least a heart rate of the person and supplying heart rate data indicative thereof. The heart rate data is supplied to a processing system. The heart rate data is processed, using a trained model implemented in the processing system, to determine the blood-alcohol content of the person.

## Description

### TECHNICAL FIELD

The present disclosure relates to blood-alcohol content detection and, more particularly, to blood-alcohol content detection using millimeter wave radar.

### BACKGROUND

Operating a vehicle, whether it be an automobile, heavy equipment, or an aircraft, under the influence of alcohol is a serious problem that can have potentially devastating consequences, such as serious injury and loss of life. In the past, several systems have been proposed for detecting vehicle operator intoxication level prior to vehicle operation. These systems include, for example, both standalone and integrated breathalyzer systems. While certainly safe and reliable, these systems do exhibit certain drawbacks. For example, these systems can be relatively expensive, intrusive, and lack public support.

Hence, there is a need for a system and method that can accurately and reliably detect the blood-alcohol content of a person that is relatively inexpensive and non-intrusive, and that, perhaps at least in part because of these factors, can garner public support. The present disclosure addresses at least these needs.

### BRIEF SUMMARY

This summary is provided to describe select concepts in a simplified form that are further described in the Detailed Description. This summary is not intended to identify key or essential features of the claimed subject matter, nor is it intended to be used as an aid in determining the scope of the claimed subject matter.

In one embodiment, a method for detecting the blood-alcohol content of a person includes detecting, using a millimeter-wave (mmWave) radar sensor, at least a heart rate of the person and supplying heart rate data indicative thereof. The heart rate data is supplied to a processing system. The heart rate data is processed, using a trained model implemented in the processing system, to determine the blood-alcohol content of the person.

In another embodiment, a system for detecting the blood-alcohol content of a person includes a millimeter-wave (mmWave) radar sensor and a processing system. The mmWave radar sensor is configured to detect at least a heart rate of the person and supply heart rate data indicative thereof. The processing system is in operable communication with the mmWave radar sensor, is coupled to receive the heart rate data and is configured, upon receipt thereof, to process the heart rate data, using a trained model implemented in the processing system, to determine the blood-alcohol content of the person.

In yet another embodiment, a method for detecting the blood alcohol content of a person includes detecting, using a millimeter-wave (mmWave) radar sensor, at least a heart rate of the person and supplying heart rate data indicative thereof. The heart rate data is supplied to a processing system. The heart rate data is processed, using a first trained model implemented in the processing system, to determine the blood-alcohol content of the person, and is processed, using a second trained model, to determine if the person is incapacitated.

Furthermore, other desirable features and characteristics of the blood alcohol-content detection system and method will become apparent from the subsequent detailed description and the appended claims, taken in conjunction with the accompanying drawings and the preceding background.

### BRIEF DESCRIPTION OF DRAWINGS

The present disclosure will hereinafter be described in conjunction with the following drawing figures, wherein like numerals denote like elements, and wherein:
FIG. 1 depicts a functional block diagram of one example embodiment of a system for detecting the blood-alcohol content of a person;
FIG.2 depicts a functional block diagram of another example embodiment of a system for detecting the blood-alcohol content of a person; and
FIG. 3 depicts one example process, in flowchart form, that may be implemented in the systems of FIGS. 1 and 2 for detecting the blood-alcohol content of a person.

### DETAILED DESCRIPTION

The following detailed description is merely exemplary in nature and is not intended to limit the invention or the application and uses of the invention. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary embodiments provided to enable persons skilled in the art to make or use the invention and not to limit the scope of the invention which is defined by the claims. Furthermore, there is no intention to be bound by any expressed or implied theory presented in the preceding technical field, background, brief summary, or the following detailed description.

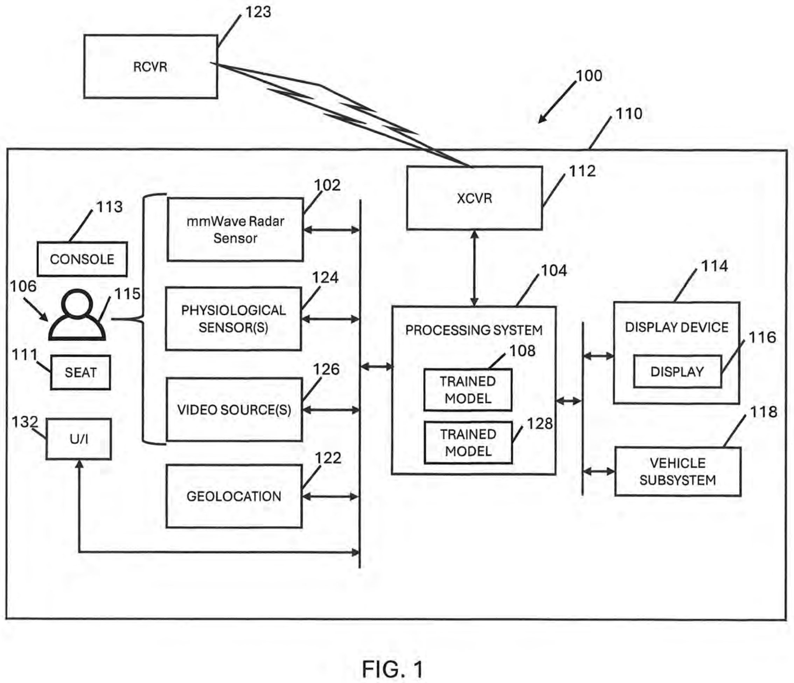

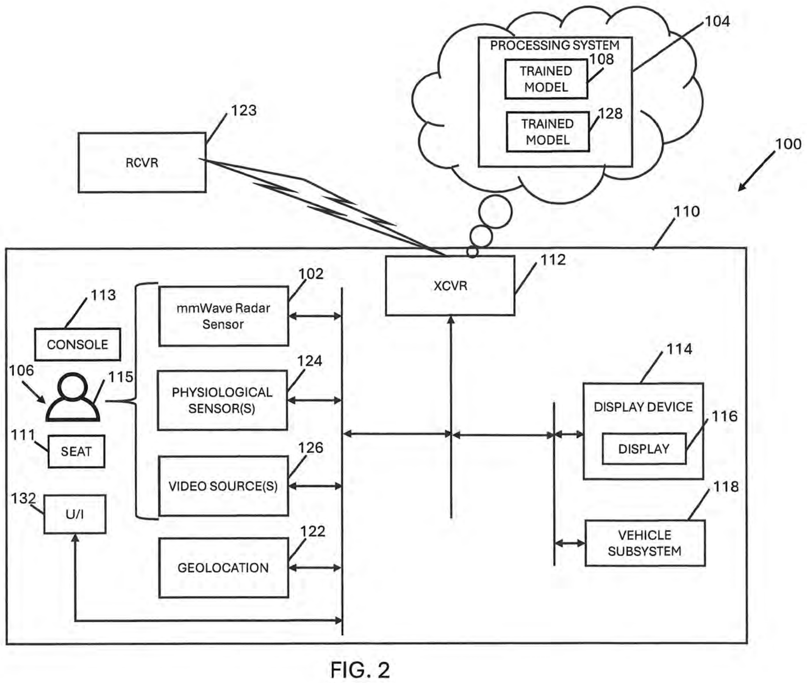

Referring to FIGS. 1 and 2, functional block diagrams of two example embodiments of a system 100 for detecting the blood-alcohol content of a person is depicted. Each of the depicted embodiments includes at least a millimeter-wave (mmWave) radar sensor 102 and a processing system 104 and are disposed at least partially within a vehicle 110. Before proceeding further, it is noted that the vehicle 110 may be any one of numerous types of mobile platforms including, for example, an automobile, a motorcycle, an aircraft, a watercraft, or a spacecraft, just to name a few.

Regardless of the specific vehicle, the mmWave radar sensor 102, as is generally known, is configured to transmit a signal having a wavelength and frequency, respectively, between 10 millimeters at 30 gigahertz and 1 millimeter at 300 GHz. When the transmitted signal is directed toward a person 106, signals are reflected from the person 106 back to the mmWave radar sensor 102. The mmWave radar sensor 102 is further configured, using any one of numerous known techniques, to process the reflected signals and thereby detect various movements in the area reflecting the transmitted signal, from which a plurality of biometric measures of the person 106 can be inferred. One of these biometric measures is the heart rate of the person 106. The mmWave radar sensor 102 is additionally configured to supply heart rate data indicative of the detected heart rate. The mmWave radar sensor 102 is preferably disposed at a location within the vehicle 110 that ensures accurate signal reflection for heart rate detection. In most, but not all embodiments, the mmWave radar sensor 102 may be disposed, for example, within a seat 111 of the vehicle 110, within a console 113 of the vehicle 110, or it may be disposed within a garment worn by the person 106.

The processing system 104 is in operable communication with the mmWave radar sensor 102. The processing system 104 may include one or more processors and computer-readable storage devices or media encoded with programming instructions for configuring the processing system 104. The one or more processors may be any custom-made or commercially available processor, a central processing unit (CPU), a graphics processing unit (GPU), an application specific integrated circuit (ASIC), a field programmable gate array (FPGA), an auxiliary processor among several processors associated with the controller, a semiconductor-based microprocessor (in the form of a microchip or chip set), any combination thereof, or generally any device for executing instructions.

The computer readable storage devices or media may include volatile and nonvolatile storage in read-only memory (ROM), random-access memory (RAM), and keep-alive memory (KAM), for example. KAM is a persistent or non-volatile memory that may be used to store various operating variables while the processor is powered down. The computer-readable storage device or media may be implemented using any of a number of known memory devices such as PROMs (programmable read-only memory), EPROMs (electrically PROM), EEPROMs (electrically erasable PROM), flash memory, or any other electric, magnetic, optical, or combination memory devices capable of storing data, some of which represent executable programming instructions, used by the controller.

With the above in mind, the processing system 104 is coupled to receive the heart rate data from the mmWave radar sensor 102. The processing system 104 is configured to process the heart rate data, using a trained model 108, to determine the blood-alcohol content of the person 106. The trained model 108, which is implemented within the processing system 104, may be a machine learning model to a statistical model that has been trained using multiple sets of data that correlates the measured blood-alcohol content of persons to their heart rate. It will be appreciated that the trained model 108 can be either a generalized model (based on data derived from multiple individuals), an individualized model (based on data derived from just the person), or a hybrid model (a generalized model updated using an individual's data). No matter the specific type of model, the data preferably is continuously (or at least periodically) updated, and the updated data is used to continuously update the trained model 108, thereby improving its accuracy over time. It should be noted that the term "blood-alcohol content" as used herein is synonymous with "blood-alcohol percentage."

It will be appreciated that the processing system 104 may be configured to process the heart rate data using any one of numerous techniques. In one particular embodiment, however, the processing system 104 is configured to implement a power spectral density (PSD) analysis technique. With this technique, the processing system 104 continuously segments the heart rate data into a predetermined number of time windows. The processing system 104 then conducts a PSD analysis of each time window to determine the power distribution in each of a plurality of different frequency bands and generates PSD data indicative of the determined power distribution. The PSD data is then supplied to the trained model 108.

No matter how the processing system 104 specifically processes the heart rate data, and as was noted above, the system 100 is at least partially disposed within the vehicle 110. For example, in some embodiments, such as the one depicted in FIG. 1, the processing system 104 is disposed entirely within the vehicle 110. However, in other embodiments, such as the one depicted in FIG. 2, the processing system 104 may be at least partially disposed remote from the vehicle 110. In such embodiments, the mmWave radar sensor 102 may be configured to wirelessly transmit the heart rate data to the processing system 104, either directly or via a transceiver 112.

As FIGS. 1 and 2 further depict, the system 100 may additionally include, at least in some embodiments, a display device 114. The display device 114, which may be implemented using any one of numerous known types of display devices, includes a display 116 and is in operable communication with the processing system 104. The display device 114 is configured, in response to image rendering display commands, to render one or more images on the display 116. The image rendering display commands are preferably supplied to the display device 114 from the processing system 104, which is further configured to command the display device 114 to render an image, on the display 116, that indicates the blood-alcohol content of the person 106. It will be appreciated that the display device 114 may be affixed to the static structure of the vehicle 110 as, for example, a Head Down Display (HDD) or Head Up Display (HUD) unit. In various embodiments, the display device 114 may assume the form of a movable display device (e.g., user-worn display device) or a portable display device, such as a laptop or a tablet computer carried into the vehicle 110.

In addition to determining the actual blood-alcohol content of the person 106, the processing system 104 is additionally configured, in at least some embodiments, to determine when the blood-alcohol content of the person 106 meets or exceeds a predetermined value. If, in fact, the blood-alcohol content of the person 106 meets or exceeds the predetermined value, the processing system 104 may additionally supply an inhibit signal to a subsystem 118 within the vehicle 110 that will at least inhibit operability of the vehicle 110. The subsystem 118 may be configured to render the vehicle 110 completely inoperable, or prevent the vehicle 110 from starting while allowing certain other vehicle functions to continue to operate, or it may be configured to take over control of the vehicle 110 and maneuver it to a safe location. As may be appreciated, the predetermined value may vary and may depend, for example, on the particular vehicle (e.g., land vehicle, watercraft, aircraft) and/or the location of the vehicle (e.g., country, state, etc.). Thus, as FIGS. 1 and 2 further depict, the system 100 may additionally include a geolocation sensor 122, such as a global positioning system (GPS) sensor, that provides location data indicative of the location of the vehicle 110.

The processing system 104 may additionally configured, in at least some embodiments, to predict, based at least in part on the determined blood-alcohol content of the person 106, the amount of time that needs to pass before blood-alcohol content of the person 106 is at or below a second predetermined value. As may also be appreciated, the second predetermined value may also vary and may depend, for example, on the particular vehicle (e.g., land vehicle, watercraft, aircraft) and/or the location of the vehicle (e.g., country, state, etc.).

In addition to indicating to the person 106, via the display device 114, the determined blood-alcohol content, it may be desired and/or required for the determined blood-alcohol content and, in at least some embodiments, vehicle location to be transmitted to various other persons and/or entities. In such embodiments, the processing system 104 is further configured to command the transceiver 112 to transmit the determined blood-alcohol content (and location) of the person 106 to one or more predetermined receivers 123 for review by another person or entity. The person or entity may be, for example, one or more persons specified by the person 106, such as a family member or friend, a law enforcement entity (police department, parole officer, etc.), or a court, just to name a few.

To further enhance the accuracy of the blood-alcohol content determination, the system 100 may include, at least in some embodiments, additional sensors. For example, as FIGS. 1 and 2 depict, the system 100 may additionally include one or more physiological sensors 124. The physiological sensors 124, when included, are in operable communication with the processing system 104, and are configured to supply one or more sets of physiological data associated with the person 106 to the processing system 104. The processing system 104 is further configured to receive and process the one or more sets of physiological data and the heart rate data, using the trained model 108, to determine the blood-alcohol content of the person. It will be appreciated that the number and type of physiological sensors 124 may vary and may include any one of numerous devices, now known or developed in the future, that sense and supply physiological data in response to physiological activity of the person 106. Some nonlimiting examples of physiological sensors 124 include oxygen saturation (SpO2) sensors, galvanic skin response sensors, breath-rate sensors, pupil diameter sensors, electroencephalogram (EEG) sensors, electromyography (EMG), and photoplethysmography (PPG), just to name a few, which may be used to determine, for example, skin conductivity, breathing rate, brain wave frequencies indicative of underlying brain activity, muscle contractions, and blood-oxygen content.

The system 100 may also include, in at least some embodiments, one or more video sources 126. The video source(s) 126, when included, is in operable communication with the processing system 104, and is configured to supply video data representative of detected video images of the person 106 to the processing system. The processing system 104 is further configured to extract a plurality of physical features from the video data, and process the extracted physical features, the one or more sets of physiological data, and the heart rate data, using the trained model 108, to determine the blood-alcohol content of the person. It will be appreciated that the physical features that the processing system 104 is configured to extract include, but are not limited to, gestures, facial expressions, and body posture. It will additionally be appreciated that the processing system 104 may implement any one or more of numerous known techniques to extract the physical features.

As FIGS. 1 and 2 depict, the processing system 104 may additionally implement a second trained model 128. When included, the processing system 104 is additionally configured to process the heart rate data, using the second trained model 128, to determine if the person 106 is incapacitated. The second trained model 128 is a model that has been trained using multiple sets of data that correlates a person's heart rate to physical incapacitation.

In some instances, the person 106 may disagree with the blood-alcohol content determined by the processing system 104 and may wish to document a dispute and/or provide other type of feedback. To facilitate this functionality, the system 100 may, at least in some embodiments, additionally include a user interface (U/I) 132 that allows the person to input their feedback and/or dispute. The feedback and/or dispute may then be transmitted, via the transceiver 112, to one or more of the predetermined receivers 123.

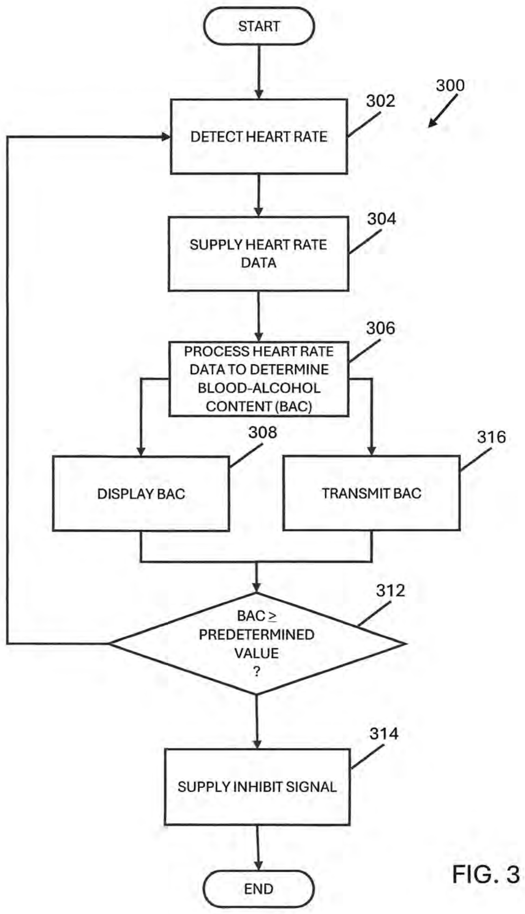

Referring now to FIG. 3, a process flowchart is depicted of one example process 300 for detecting the blood-alcohol content of a person. The order of operation within the process 300 is not limited to the sequential execution as illustrated in the figure but may be performed in one or more varying orders as applicable and in accordance with the present disclosure. Moreover, as will be explained further below, some of the depicted steps may not be performed at all.

The process 300 begins by detecting, using the mmWave radar sensor 102, the heart rate of the person 106 (302) and supplying heart rate data indicative the heart rate to a processing system 104 (304). The heart rate data is then processed, using the trained model 108 implemented in the processing system, to determine the blood-alcohol content of the person 106 (306). Although the process 300 implemented in the system 100 may not, in at least some embodiments, include additional steps, the depicted process 300 does include additional steps, which will now be described.

After determining the blood-alcohol content of the person 106, the processing system 104 may also command the display device 114 to render an image on the display 116 that indicates the blood-alcohol content of the person 106 (308). The processing system 104 may additionally determine if the blood-alcohol content of the person meets or exceeds a predetermined value (312). If not, then the process repeats. If so, however, the processing system 104 supplies an inhibit signal to the subsystem 118 within the vehicle 110 that will at least inhibits operability of the vehicle 110 (314). The processing system 104 may also be configured to transmit the determined blood-alcohol content of the person 106 to one or more predetermined receivers for review by another person 106 (316).

The system and method described herein can accurately and reliably detect the blood-alcohol content of a person and do so in a manner that is relatively inexpensive and non-intrusive.

Those of skill in the art will appreciate that the various illustrative logical blocks, modules, circuits, and algorithm steps described in connection with the embodiments disclosed herein may be implemented as electronic hardware, computer software, or combinations of both. Some of the embodiments and implementations are described above in terms of functional and/or logical block components (or modules) and various processing steps. However, it should be appreciated that such block components (or modules) may be realized by any number of hardware, software, and/or firmware components configured to perform the specified functions. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention. For example, an embodiment of a system or a component may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments described herein are merely exemplary implementations.

The various illustrative logical blocks, modules, and circuits described in connection with the embodiments disclosed herein may be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor. The processor and the storage medium may reside in an ASIC.

Techniques and technologies may be described herein in terms of functional and/or logical block components, and with reference to symbolic representations of operations, processing tasks, and functions that may be performed by various computing components or devices. Such operations, tasks, and functions are sometimes referred to as being computer-executed, computerized, software-implemented, or computer-implemented. In practice, one or more processor devices can carry out the described operations, tasks, and functions by manipulating electrical signals representing data bits at memory locations in the system memory, as well as other processing of signals. The memory locations where data bits are maintained are physical locations that have particular electrical, magnetic, optical, or organic properties corresponding to the data bits. It should be appreciated that the various block components shown in the figures may be realized by any number of hardware, software, and/or firmware components configured to perform the specified functions. For example, an embodiment of a system or a component may employ various integrated circuit components, e.g., memory elements, digital signal processing elements, logic elements, look-up tables, or the like, which may carry out a variety of functions under the control of one or more microprocessors or other control devices.

When implemented in software or firmware, various elements of the systems described herein are essentially the code segments or instructions that perform the various tasks. The program or code segments can be stored in a processor-readable medium or transmitted by a computer data signal embodied in a carrier wave over a transmission medium or communication path. The "computer-readable medium", "processor-readable medium", or "machine-readable medium" may include any medium that can store or transfer information. Examples of the processor-readable medium include an electronic circuit, a semiconductor memory device, a ROM, a flash memory, an erasable ROM (EROM), a floppy diskette, a CD-ROM, an optical disk, a hard disk, a fiber optic medium, a radio frequency (RF) link, or the like. The computer data signal may include any signal that can propagate over a transmission medium such as electronic network channels, optical fibers, air, electromagnetic paths, or RF links. The code segments may be downloaded via computer networks such as the Internet, an intranet, a LAN, or the like.

Some of the functional units described in this specification have been referred to as "modules" in order to more particularly emphasize their implementation independence. For example, functionality referred to herein as a module may be implemented wholly, or partially, as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices, or the like. Modules may also be implemented in software for execution by various types of processors. An identified module of executable code may, for instance, comprise one or more physical or logical modules of computer instructions that may, for instance, be organized as an object, procedure, or function. Nevertheless, the executables of an identified module need not be physically located together, but may comprise disparate instructions stored in different locations that, when joined logically together, comprise the module and achieve the stated purpose for the module. Indeed, a module of executable code may be a single instruction, or many instructions, and may even be distributed over several different code segments, among different programs, and across several memory devices. Similarly, operational data may be embodied in any suitable form and organized within any suitable type of data structure. The operational data may be collected as a single data set, or may be distributed over different locations including over different storage devices, and may exist, at least partially, merely as electronic signals on a system or network.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order without departing from the scope of the invention as long as such an interchange does not contradict the claim language and is not logically nonsensical.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

As used herein, the term "axial" refers to a direction that is generally parallel to or coincident with an axis of rotation, axis of symmetry, or centerline of a component or components. For example, in a cylinder or disc with a centerline and generally circular ends or opposing faces, the "axial" direction may refer to the direction that generally extends in parallel to the centerline between the opposite ends or faces. In certain instances, the term "axial" may be utilized with respect to components that are not cylindrical (or otherwise radially symmetric). For example, the "axial" direction for a rectangular housing containing a rotating shaft may be viewed as a direction that is generally parallel to or coincident with the rotational axis of the shaft. Furthermore, the term "radially" as used herein may refer to a direction or a relationship of components with respect to a line extending outward from a shared centerline, axis, or similar reference, for example in a plane of a cylinder or disc that is perpendicular to the centerline or axis. In certain instances, components may be viewed as "radially" aligned even though one or both of the components may not be cylindrical (or otherwise radially symmetric). Furthermore, the terms "axial" and "radial" (and any derivatives) may encompass directional relationships that are other than precisely aligned with (e.g., oblique to) the true axial and radial dimensions, provided the relationship is predominantly in the respective nominal axial or radial direction. As used herein, the term "substantially" denotes within 5% to account for manufacturing tolerances. Also, as used herein, the term "about" denotes within 5% to account for manufacturing tolerances.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth in the appended claims.

## Claims

1. A method for detecting a blood-alcohol content of a person, the method comprising the steps of:
detecting, using a millimeter-wave (mmWave) radar sensor, at least a heart rate of the person and supplying heart rate data indicative thereof;
supplying the heart rate data to a processing system;
processing the heart rate data, using a trained model implemented in the processing system, to determine the blood-alcohol content of the person.

2. The method of claim 1, wherein the step of processing the heart rate data comprises:
continuously segmenting the heart rate data, in the processing system, into a predetermined number of time windows;
conducting a power spectral density (PSD) analysis of each time window, in the processing system, to a determine power distribution in each of a plurality of different frequency bands and generate PSD data indicative thereof; and
supplying the PSD data to the trained model.

3. The method of claim 1, further comprising:
commanding a display device, using the processing system, to render an image on a display that indicates the blood-alcohol content of the person.

4. The method of claim 1, further comprising:
determining, in the processing system, when the blood-alcohol content of the person meets or exceeds a predetermined value; and
when the blood-alcohol content of the person meets or exceeds the predetermined value, supplying an inhibit signal, from the processing system to a subsystem within a vehicle, that will at least inhibit operability of the vehicle.

5. The method of claim 1, further comprising:
transmitting the determined blood-alcohol content of the person to one or more predetermined receivers for review by another person.

6. The method of claim 1, wherein:
the processing system is located remote from the mmWave radar sensor; and
the step of supplying the heart rate data comprises wirelessly transmitting the heart rate data to the processing system.

7. The method of claim 1, further comprising:
sensing, using one or more physiological sensors, one or more sets of physiological data associated with the person;
supplying the one or more sets of physiological data to the processing system; and
processing the one or more sets of physiological data and the heart rate data, using the trained model implemented in the processing system, to determine the blood-alcohol content of the person.

8. The method of claim 7, further comprising:
processing, in the processing system, video data supplied from a video source to extract a plurality of physical features from the video data, the video data being representative of detected video images of the person; and
processing the extracted physical features, the one or more sets of physiological data, and the heart rate data, using the trained model implemented in the processing system, to determine the blood-alcohol content of the person.

9. The method of claim 1, wherein the processing system is further configured to predict, based at least in part on the blood-alcohol content of the person, an amount of time until the person is not intoxicated.

10. A system for detecting a blood-alcohol content of a person, comprising:
a millimeter-wave (mmWave) radar sensor configured to detect at least a heart rate of the person and supply heart rate data indicative thereof; and
a processing system in operable communication with the mmWave radar sensor, the processing system coupled to receive the heart rate data and configured, upon receipt thereof, to process the heart rate data, using a trained model implemented in the processing system, to determine the blood-alcohol content of the person.

11. The system of claim 10, wherein the processing system is configured to process the heart rate data by:
continuously segmenting the heart rate data into a predetermined number of time windows;
conducting a power spectral density (PSD) analysis of each time window to a determine power distribution in each of a plurality of different frequency bands and generate PSD data indicative thereof; and
supplying the PSD data to the trained model.

12. The system of claim 10, further comprising:
a display device in operable communication with the processing system, the display device including an display and configured, in response to image rendering display commands, to render one or more images on the display,
wherein the processing system is further configured to command the display device to render an image on the display that indicates the blood-alcohol content of the person.

13. The system of claim 10, wherein the processing system is further configured to:
determine when the blood-alcohol content of the person meets or exceeds a predetermined value; and
when the blood-alcohol content of the person meets or exceeds the predetermined value, supply an inhibit signal to a subsystem within a vehicle that will at least inhibit operability of the vehicle.

14. The system of claim 10, further comprising:
a transmitter in operable communication with the processing system,
wherein the processing system is further configured to command the transmitter to transmit the determined blood-alcohol content of the person to one or more predetermined receivers for review by another person.

15. The system of claim 10, wherein:
the processing system is located remote from the mmWave radar sensor; and
the mmWave radar sensor is configured to wirelessly transmit the heart rate data to the processing system.
